# EUROPÄISCHE PATENTSCHRIFT

(11) **EP 2 098 255 B1**
(45) Veröffentlichungstag und Bekanntmachung des Hinweises auf die Patenterteilung: **03.12.2014**
(21) Anmeldenummer: 09003282.2
(22) Anmeldetag: 06.03.2009
(51) Int. Cl.: A61L 27/24, A61L 26/00

(54) **Verfahren zur Herstellung von Kollagen-material**
Method for producing collagen material
Procédé de fabrication de matériau collagène

(30) Priorität: 07.03.2008 DE 102008013091; 01.07.2008 DE 202008008627 U
(43) Veröffentlichungstag der Anmeldung: 09.09.2009
(73) Patentinhaber: aap Biomaterials GmbH, 64807 Dieburg (DE)
(72) Erfinder: Heimann, Lydia, 63768 Hösbach (DE); Dingeldein, Elvira, Dr., 64287 Darmstadt (DE); Voges, Michael, 64739 Höchst (DE); Schubert, Ellen, 63834 Sulzbach (DE)
(74) Vertreter: Herden, Andreas F.

(56) Entgegenhaltungen:
- EP-A2- 0 901 795
- WO-A1-90/00060
- DE-A1- 3 203 957

## Beschreibung

### Gebiet der Erfindung

Die Erfindung betrifft ein Verfahren zur Herstellung von Kollagen-Material, insbesondere in Form eines schwammartigen und/oder vliesartigen Körpers, ein nach dem Verfahren hergestelltes Kollagen-Material und die Verwendung des nach dem Verfahren hergestellten Kollagen-Materials.

### Hintergrund der Erfindung

Kollagen ist das Gerüsteiweiß des Bindegewebes in Faserform. Je nach Struktur, pharmakologischer Wirkung und Art der Herkunft unterscheidet man zwischen Kollagen vom Typ I, II, III, X usw. Kollagen-Fasern bzw. -Fibrillen werden im Wesentlichen aus zerkleinerten Achillessehnen oder aus Unterhautgewebe von Rindern und Pferden gewonnen. Aufgrund der fortbestehenden BSE-Problematik wird heute Kollagen bovinen Ursprungs praktisch nicht mehr verwendet. Kollagen wird durch schonende Hydrolyse gewonnen. Es handelt sich um ein hochmolekulares Protein und eine physiologische Substanz.

Das nach bekannten Verfahren gewonnene Rohmaterial durchläuft verschiedene Reinigungs- und Aufbereitungsschritte und wird schließlich üblicherweise einer Lyophilisation unterzogen, wobei das Endprodukt in Form hochporöser, fasriger Platten anfällt, die in der Größe von einigen Quadratzentimetern (z.B. 5x5, 5x10, 10x10 cm) einer Dicke von ca. 3 bis 6 mm und einem Gewicht von ca. 50 bis 250 g/m², bevorzugt von 70 bis 130 g/m², in den Handel kommen.

Üblicherweise erfolgt die Sterilisation durch Behandlung mit γ-Strahlen. Die handelsüblichen Kollagen-Schwämme enthalten meist noch ein Antibiotikum zur Infektionsprophylaxe, wobei insbesondere Gentamicin, aber auch Vancomycin zur Anwendung kommen. Der Gentamicingehalt beträgt beispielsweise 1,3 mg Base, entsprechend 2 mg Sulfat pro cm². Um die Stabilität der Schwämme, die im nassen Zustand kollabieren, quellen und sich auflösen, zu verbessern, wird ggf. eine Quervernetzung der Fasern durch eine chemische Behandlung mit z.B. Glutar- oder Formaldehyd durchgeführt.

Diese Behandlung ist jedoch aus toxikologischer Sicht nicht unbedenklich. Außerdem beeinträchtigt sie die hämostyptische Wirkung und verringert die Resorption. Die Resorption erfolgt durch die Aktivität eingewanderter Makrophagen und durch Kollagenasen. In Abhängigkeit vom Vernetzungsgrad wurden beim Löslichkeitsverhalten verschiedene Halbwertszeiten ermittelt. Im Tierversuch wurde nach s.c. Implantation von Rinderkollagen (Schwämmen) in Ratten eine Resorptionszeit von über 40 Tagen beobachtet. In der Knochenchirurgie bei Kaninchen betrug die Resorptionszeit von Kollagen-Schwämmen über 3 bis 6 Wochen (Monographie: Kollagen, tierischer Herkunft, Bundesanzeiger Nr. 149, 10. Aug. 94). Grundsätzlich wird die Kollagenresorption vom Ort der Applikation, von Menge und Art des Kollagen-Implantates sowie einer vorhandenen natürlichen Quervernetzung oder Aldehydvernetzung beeinflusst.

Wesentliche klinische Anwendungsgebiete für Kollagen-Schwämme sind u.a. die Verwendung als Wundauflagen zur lokalen Blutstillung, die Auskleidung von Gewebedefekten, der Hautersatz bei Läsionen, die Hautabdeckung bei großflächigen Verbrennungen, die Füllung von Knochendefekten z.B. nach Zystektomien, auch im Kieferbereich.

Die hämostyptischen (blutstillenden) Eigenschaften des aus tierischer Haut gewonnenen Kollagens (Typ I) werden durch den Kontakt der Thrombozyten des Blutes mit der nativen tripelhelikalen Struktur des Kollagens bei gleichzeitiger Aktivierung der Blutgerinnung erklärt. Wesentlich für die Blättchenaggregation ist die Fibrillenstruktur des nativen Kollagens und sein polarer Molekülaufbau. Für Verfahren zur Herstellung von solchen speziell als Hämostypika bestimmten Kollagen-Produkten wurde als Ausgangsrohstoff auch bereits porcines Material, nämlich Schweinehaut (z.B. DD 233 785 A1 bzw. DD 292 840 A5) beschrieben.

Bekannte Hämostypika aus nativem Kollagen weisen zumeist einen sehr niedrigen pH-Wert, typischerweise einen pH-Wert unter 6 auf. Hierdurch können Wundheilungsstörungen auftreten.

Die europäische Patentschrift EP 428541 B1 zeigt dagegen die Herstellung eines Kollagenimplantats mit höherem pH-Wert. Hierfür wird ein Säurepuffer zugesetzt. Zur Herstellung des Kollagenimplantats wird allerdings kein natives Kollagen-Ausgangsmaterial sondern ein Atelokollagen verwendet. Im Unterschied zu nativen Kollagenausgangsstoffen sind Atelokollagene gut löslich und bei der Weiterverarbeitung unempfindlich gegenüber pH-Werten von mehr als 6,5. Trotz des hohen pH-Wertes verfestigt sich eine Atelokollagenlösung relativ einfach. Bei der Verarbeitung von nativem Kollagen stabilisiert dagegen die Säure die Kollagenstruktur, vermutlich durch Denaturierung der Eiweißstränge. Bei höheren pH-Werten tritt dieser Effekt nicht mehr auf, so dass die Struktur zerfällt. Gegenüber Telopeptid-haltigen Kollagen, also Kollagenen, bei denen die Telopeptide nicht chemisch entfernt wurden, besitzen Atelokollagene eine weniger gute blutstillende Wirkung. Weiter sind aus Atelokollagenen hergestellte Implantate im trocknen Zustand in der Regel spröde und wenig biegefest. Im nassen Zustand hingegen sind die aus Atelokollagenen hergestellten Implantate in der Regel nicht mehr formstabil und besitzen nur noch eine sehr geringe Reißfestigkeit, so dass ein einmal feucht gewordenes Kollagenimplantat nicht mehr von der Wunde abgezogen und versetzt werden kann.

Das Dokument EP 0 901 795 A2 zeigt die Herstellung eines bioabsorbierbaren Materials, welches unter Verwendung eines Natriumhydrogenphosphatpuffers auf einen pH-Wert zwischen 4,5 und 6,5 eingestellt wird. Verwendet wird ein durch enzymatischen Abbau gewonnenes Kollagenmaterial.

Trotz ihrer häufigen Anwendung in Chirurgie und Orthopädie und verschiedener im Laufe der Jahre durchgeführter Änderungen im Herstellungsverfahren, haben die im Markt befindlichen Produkte, die vorwiegend zur Blutstillung nach chirurgischen Eingriffen zur Anwendung kommen, erhebliche Nachteile, die sowohl die Handhabung als auch das biologische Verhalten betreffen.

So zeigt sich in der Praxis, dass die Kollagen-Materialien, beispielsweise in Form von Schwämmen, oft eine zu geringe Nassfestigkeit besitzen, eine zu lange Resorptionszeit aufweisen, an den chirurgischen Instrumenten und Handschuhen haften und Unverträglichkeitserscheinungen bewirken, wobei zum Teil starke Serombildung und Wundheilungsstörungen im Vordergrund stehen.

Die Nassfestigkeit ist deshalb von Bedeutung, weil unmittelbar bei der Applikation der Kollagen-Materialien diese sehr rasch Gewebsflüssigkeit und insbesondere Blut aufnehmen, damit kollabieren und ihre schwammige Struktur und Festigkeit verlieren. Dies hat zur Folge, dass dann das Material auf Wundflächen nicht mehr korrekt plaziert werden kann und somit oft notwendige Lagekorrekturen am Applikationsort in der Wunde nicht mehr möglich sind.

Darüber hinaus haben die Kollagen-Materialien oft die Eigenschaft, an den chirurgischen Instrumenten (Scheren, Klemmen, Pinzetten usw.) bzw. Handschuhen zu haften, sobald sie feucht sind, was eine korrekte zielgenaue Plazierung in der Wunde außerordentlich erschwert. Insbesondere bei den immer häufiger praktizierten Verfahren der minimalinvasiven Chirurgie erweist sich diese Eigenschaft als außergewöhnlich störend und hinderlich, da bei diesen Eingriffen nur eine sehr geringe räumliche Bewegungsfreiheit gegeben ist.

Von großem Nachteil ist die lange Resorptionszeit von ca. 20-40 Tagen. Bestimmungsgemäß ist der bevorzugte Anwendungszweck, die Blutstillung, nämlich nach wenigen Minuten bis Stunden erreicht und damit der eigentliche Zweck der Applikation erfüllt. Auch das zur Infektionsprophylaxe zugesetzte Antibiotikum wird in der Regel sehr rasch aus dem Kollagen-Material eluiert, so dass ein Antibiotikumschutz nur für wenige Tage gewährleistet ist. Danach stellt das Kollagen-Material bei einer verzögerten bakteriellen Besiedlung eher eine Gefahr dar, da dann das antibiotikumfreie Kollagen in der Wunde als Nährboden für Infektionserreger dienen kann, insbesondere dann, wenn wegen einer starken Sekretion die Wunde drainiert werden muss. Aus diesen Gründen wäre daher eine sehr viel kürzere Verweildauer (Resorptionszeit) des Kollagen-Materials außerordentlich wünschenswert.

Der entscheidende medizinische Nachteil der marktüblichen Kollagen-Materialien beruht aber auf den häufig zu beobachtenden teilweise sehr ausgeprägten Unverträglichkeitserscheinungen. Diese führen sehr bald nach der Applikation zu entzündlichen Prozessen mit Zellnekrosen und einer starken Wundsekretion (Serombildung), die den natürlichen Heilungsprozess empfindlich stört und stark verzögert. Oft wird dadurch ein regulärer Wundverschluss verhindert. Diese Erscheinungen führen immer häufiger dazu, dass Chirurgen und Orthopäden auf die an sich gewünschte und indizierte Anwendung von Kollagen-Materialien verzichten, da die beschriebenen Nebenwirkungen aus ärztlicher Sicht nicht in Kauf genommen werden können und auch dem Patienten ein gestörter und verzögerter Wundheilungsverlauf nicht zuzumuten ist.

### Aufgabe der Erfindung

Der Erfindung liegt die Aufgabe zugrunde, ein besonders wirtschaftliches Verfahren zur Herstellung eines Kollagen-Materials, insbesondere in Form eines schwammartigen und/oder vliesartigen Körpers, anzugeben, welches/r in der Anwendung und Handhabung einfacher und sicherer, in der Resorptionszeit deutlich kürzer und in der Verträglichkeit nach Möglichkeit wesentlich besser als die zur Zeit verfügbaren Produkte ist. Das heißt, das neu zu entwickelnde Kollagen-Material sollte eine gute hämostyptische Wirkung zeigen, eine verbesserte Nassfestigkeit besitzen, besser handhab- und applizierbar sein, nicht an Instrumenten und dergleichen haften, eine kürzere Resorptionszeit aufweisen und eine wesentlich verbesserte Verträglichkeit besitzen, damit es in der Human- und/oder Veterinärmedizin und insbesondere zur Wundabdeckung oder als Implantat Verwendung finden kann.

Insbesondere ist eine Aufgabe der Erfindung, ein Kollagen-Material bereitzustellen, welches sowohl im nassen als auch im trockenen Zustand formstabil und biegefest ist und allenfalls ein schwach sauren pH-Wert aufweist.

### Zusammenfassung der Erfindung

Die Aufgabe der Erfindung wird bereits durch ein Verfahren zur Herstellung eines Kollagen-Materials sowie durch ein kollagenhaltiges Flächengebilde nach einem der unabhängigen Ansprüche gelöst.

Bevorzugte Ausführungsformen und Weiterbildungen der Erfindung sind den jeweiligen Unteransprüchen zu entnehmen.

Gemäß der Erfindung ist zum einen ein Verfahren zur Herstellung eines Kollagen-Materials, insbesondere eines Implantats zur Wundabdeckung vorgesehen.

Dazu wird eines Suspension hergestellt, welche ein Telopeptid-haltiges Kollagen umfasst. Es wird also kein chemisch behandeltes Atelokollagen verwendet, bei welchem die Telopeptidketten größtenteils entfernt sind und welches sich relativ gut in Wasser löst.

Vielmehr wird zur Herstellung vorzugsweise ein natives Kollagen verwendet, aus dem vorzugsweise unter Zugabe einer Säure eine kollagenhaltige Suspension erzeugt wird. Das Kollagen wird somit nicht aufgelöst, sondern bleibt in partikulärer Form in der Suspension erhalten.

Der Suspension wird ein Phosphatpuffer zugegeben, wodurch bereits die Suspension auf einen in etwa neutralen PH-Wert eingestellt werden kann.

Die Erfinder haben herausgefunden, dass die Verwendung eines Phosphatpuffers zu einer Verfestigung des Kollagen-Materials führt. Vermutlich kommt diese Verfestigung durch eine Vernetzung der Kollagenfibrillen, beispielsweise aufgrund Van-der-Waals-Kräfte zustande. Hierzu wird die Suspension ruhen gelassen, bis die Vernetzung beziehungsweise Verfestigung hinreichend fortgeschritten ist.

Sodann kann ein Flächengebilde durch Trocknung des entstandenen kollagenhaltigen Gemisches hergestellt werden. Unter Trocknung wird jeder Vorgang verstanden, bei welchem das Wasser entfernt wird, und das Kollagen sowie weitere Feststoffe in Form eines Flächengebildes zurückbleiben.

Vorzugsweise erfolgt die Trocknung im Rahmen eines Gefriertrocknungsprozesses, wobei beispielsweise das kollagenhaltige Gemisch in flachen Schalen schockgefroren und sodann im Vakuum getrocknet wird.

Bei einer bevorzugten Ausführungsform der Erfindung wird die Suspension unter Zugabe von Phosphorsäure hergestellt. Hierdurch wird der Phosphatanteil des entstandenen Gemisches nach Zugabe des Phosphatpuffers erhöht, wodurch die Festigkeit des entstandenen Flächengebildes erhöht wird.

Die Zugabe der Säure im Allgemeinen scheint zumindest zum Aufquellen der Kollagene zu führen, wodurch sich zumindest teilweise Kollagenfibrillen bilden, welche nach Zugabe des Phosphatpuffers miteinander vernetzen können.

Bei einer bevorzugten Ausführungsform der Erfindung wird durch den Phosphatpuffer die Suspension auf einen pH-Wert zwischen 5,5 und 8,5, bevorzugt zwischen 5,8 und 7,8 und besonders bevorzugt zwischen 6,0 und 7,0 eingestellt.

Es hat sich herausgestellt, dass bei pH-Werten von unter 6,0 die Wundheilung spürbar verschlechtert ist. pH-Werte von über 7,0 können dagegen zu Veränderungen, insbesondere zugesetzter Antibiotika führen. Insbesondere zugesetzte Gentamicine können Toxine bilden.

Als Phosphatpuffer wird vorzugsweise ein NatriumhydrogenPhosphat, insbesondere Di- oder Trinatriumhydrogenphosphat verwendet. Die Natriumionen führen im Gegensatz zu den Ionen anderer Phosphate zu keinen unerwünschten Wechselwirkungen mit dem Gewebe.

Bei einer bevorzugten Ausführungsform der Erfindung wird eine Suspension mit einem Kollagengehalt von 0,3 bis 7, vorzugsweise von 0,5 bis 3, besonders bevorzugt von 0,6 bis 1,5 Gew.% hergestellt. Vor der Trocknung beträgt bei einer bevorzugten Ausführungsform der Erfindung der Feststoffgehalt des entstandenen Gemisches zwischen 0,2 bis 10 Gew.% , bevorzugt 0,3 bis 8 Gew.% und besonders bevorzugt 0,5 bis 2,5 Gew.% . Der Feststoffgehalt ist der Anteil der nach einer Trocknung des Gemisches verbleibenden Feststoffe.

Insbesondere über den Kollagengehalt der Suspension, den Anteil des Kollagens und den Feststoffgehalt kann das Flächengewicht und die Porosität des entstehenden Materials eingestellt werden.

Mit der Erfindung lässt sich ein Flächengebilde bereitstellen, welches einen Phosphat-, insbesondere einen Dinatriumhydrogen-Phosphatanteil von 80 bis 500, vorzugsweise von 100 bis 400 und besonders bevorzugt von 220 bis 370 mg/g aufweist. Die Phosphatanteile in dem genannten Bereich sind besonders gewebeverträglich.

Bei einer Weiterbildung der Erfindung wird die Suspension mechanisch denaturiert, insbesondere in einer Kolloidmühle. Eine derartige mechanische Denaturierung, bei welcher lediglich eine Säure zugesetzt ist, führt dazu, dass die Eigenschaften des nativen Kollagens weitgehend erhalten bleiben, wodurch insbesondere die blutstillenden Eigenschaften gegenüber bekannten Implantaten aus Atelokollagenen verbessert sind.

Mit der Erfindung können relativ dicke Flächengebilde mit einer Dicke zwischen 1 und 10 mm, bevorzugt zwischen 2 und 7 mm und einem recht geringen Flächengewicht, vorzugsweise zwischen 20 und 200, besonders bevorzugt zwischen 50 und 150 g/qm hergestellt werden.

So kann ein Flächengebilde bereitgestellt werden, welches mindestens das Dreifache, vorzugsweise mindestens das Fünffache und besonders bevorzugt mindestens das Achtfache seines Eigengewichtes an Flüssigkeit, insbesondere an Wasser aufnimmt.

Als Ausgangsmaterial zur Herstellung des Kollagen-Materials werden vorzugsweise Schweinehäute verwendet. Im Vergleich zu bovinen oder equinen Geweben besitzen porcine Kollagene, insbesondere wenn sie nicht stark chemisch verändert werden, eine bessere Verträglichkeit.

Bei einer Weiterbildung der Erfindung wird dem Kollagen-Material ein Antibiotikum zugesetzt. Insbesondere wird in die Suspension ein Antibiotikum wie Gentamicin, insbesondere Gentamicin-Sulfat und/oder Gentamicin-Phosphat eingerührt.

Gentamicin-Sulfat ist ein Standard-Antibiotikum mit recht guter Verträglichkeit. Gegenüber Gentamicin-Sulfat hat die Beigabe von Gentamicin-Phosphat aber den Vorteil, dass die Phosphatkonzentration erhöht wird, wodurch wiederum die Festigkeit des hergestellten Flächenmaterials verbessert wird. So kann bei Verwendung von Gentamicin-Phosphat auch der Anteil des Phosphatpuffers reduziert werden.

Mit der Erfindung lässt sich ein nass-stabiles und auch im trockenen Zustand biegefestes poröses Flächengebilde bereitstellen, welches einen pH-Wert von mehr als 5,8, vorzugsweise mehr als 6,5, besonders bevorzugt mehr als 7,2 aufweist. Der pH-Wert des Flächengebildes im Sinne der Erfindung wird durch Tränken des Flächengebildes mit deionisiertem Wasser und anschließendes Messen des pH-Wertes bestimmt.

Dadurch, dass das Flächengebilde Kollagene umfasst, welche zumindest teilweise Telopeptide umfassen, ist ferner eine besonders gute Blutstellung sichergestellt.

Vorzugsweise beträgt der Anteil von telopeptidhaltigem Kollagen mehr als 40 %, bevorzugt mehr als 60 % und besonders bevorzugt mehr als 80 %.

Vorzugsweise werden zur Herstellung des kollagenhaltigen Flächengebildes nur native Kollagene verwendet. Der Kollagengehalt des Flächengebildes beträgt zwischen 10 und 80, vorzugsweise zwischen 20 und 60 Gew.%.

Das Flächengebilde hat eine Nass-Reißfestigkeit von mehr als 0,05, vorzugsweise von mehr als 0,07, besonders bevorzugt mehr als 0,1 N/mm². So lässt sich das erfindungsgemäße Implantat auch nach Aufsetzen auf eine Wunde abnehmen und verschieben.

Bezogen auf einen 1 cm breiten Streifen des Flächengebildes beträgt die Nass-Reißfestigkeit mehr als 0,5, vorzugsweise mehr als 1,0, besonders bevorzugt mehr als 1,5 N/cm.

### Beschreibung bevorzugter Ausführungsbeispiele

Ein kollagenhaltiges Flächengebilde kann beispielsweise wie folgt hergestellt werden:

Ausgehend von einem porcinen Ausgangsmaterial wird Schweinehaut basisch und oxidativ aufgereinigt und anschließend mit verdünnter Phosphorsäure sauer eingestellt, worauf die Haut gespült, zerkleinert und nach Zwischenfrostung bei -28 ± 10 °C mit Wasser vermischt wird, so dass eine homogene Suspension mit einem Kollagengehalt von etwa 1 % entsteht, die Suspension wird gegebenenfalls mit wenigstens einem Antibiotikum verrührt und das gesamte Gemisch dann durch Zugabe eines Phosphatpuffers auf neutralen bis leicht basischen pH-Wert von bevorzugt 6,5 bis 8,5, besonders bevorzugt 7,2 bis 7,8, eingestellt, der Feststoffgehalt wird auf 0,5 bis 2,5 % eingestellt und das Gemisch nach einsetzender oder erfolgreicher Fibrillierung der Suspension lyophilisiert (gefriergetrocknet).

So wird als Ausgangsmaterial für die erfindungsgemäße Kollagengewinnung Schweinehaut verwendet, so dass einerseits die bei bovinen Rohstoffen bestehende BSE-Problematik sicher ausgeschlossen werden kann. Andererseits ist bekannt, dass die biologischen Eigenschaften von Schweinegeweben, insbesondere von Schweinehaut, denen menschlicher Organe, insbesondere der Haut, näher stehen als bovine oder equine Gewebe.

Bei dem Verfahren ist im Hinblick auf die Stabilität des herzustellenden Kollagen-Materials von besonderer Bedeutung, dass die oxidative Aufbereitung der Schweinehaut nicht im sauren, sondern im neutralen bis basischen Milieu erfolgt. Eine oxidative Behandlung, insbesondere mit Wasserstoffperoxid, nach der sauren Behandlung wirkt sich nämlich negativ auf die zur Stabilisierung des Kollagen-Materials notwendige Fibrillierung im physiologischen pH-Bereich von 7,2 bis 7,8 aus und wird daher vor der sauren Behandlung durchgeführt.

Es wurde ferner gefunden, dass die Säurebehandlung mit Phosphorsäure für die Stabilität, insbesondere aber für die Kollagenhaftung an Instrumenten und dergleichen von entscheidender Bedeutung ist. So kann überraschenderweise insbesondere das fehlende Anhaften an Instrumenten erfindungsgemäß dadurch erreicht werden, dass im Herstellungsprozess die üblicherweise durchgeführte Salzsäurebehandlung durch eine Anwendung von Phosphorsäure ersetzt wird.

Bedeutsam ist ferner, dass die Haut erst nach der Zwischenfrostung mit Wasser vermischt wird, um einen Kollagengehalt von etwa 0,5 bis 3,5 % einzustellen. Durch einen Überschuss an Wasser bei der Zwischenfrostung wird nämlich die Kollagenstruktur geschädigt, so dass im physiologischen pH-Bereich am Ende der Kollagen-Material-Herstellung nicht die gewünschte Stabilität erreicht wird. Daher wird die Zwischenfrostung im Sauren, bevorzugt bei einem pH-Wert von 3,0 ± 0,5, durchgeführt.

Für die Verbesserung der Nassfestigkeit und Elastizität des Kollagen-Materials erweist sich der höhere pH-Wert von 5,5 bis 8,5, bevorzugt von 6,0 bis 8,0, besonders bevorzugt von 6,3 bis 7,7, durch die Phosphatpufferung nach Zugabe oder besonders bevorzugt mit Zugabe des Antibiotikums als positiv, da dadurch eine bessere Fibrillierung erreicht werden konnte.

Durch das Verrühren der Suspension mit dem Antibiotikum in Form einer phosphatgepufferten Lösung bevorzugt mit einem pH-Wert von 6,5 bis 7,8 kann auf eine zusätzliche Verstärkung der Vernetzung, beispielsweise durch eine mechanische Behandlung, insbesondere eine Rüttlungsbehandlung, mit einer anschließenden mehrstündigen Ruhigstellung verzichtet werden.

Vorteilhafterweise wird die Suspension mit dem Antibiotikum als Phosphatlösung eingesetzt, d.h. als Gegenion des Antibiotikums wird Phosphat eingesetzt. Damit sind in der Suspension keine störenden Anionen mehr vorhanden, wodurch eine zusätzliche Verstärkung der Vernetzung, beispielsweise durch eine mechanische Behandlung, insbesondere eine Rüttlungsbehandlung, mit einer anschließenden mehrstündigen Ruhigstellung, nicht notwendig ist.

Bevorzugt stammt die verwendete Schweinehaut ausschließlich von jungen, 6 bis 9 Monate alten Schlachttieren. Diese stellt nämlich ein homogeneres und somit biologisch wertvolleres Ausgangsmaterial dar als dies bei Rindern oder Pferden der Fall ist, die erst in höherem Alter zur Schlachtung kommen.

Bei der Aufbereitung der Schweinehaut hat sich überraschend gezeigt, dass für wesentliche Eigenschaften des Endproduktes und zwar u.a. der Elastizität, der mechanischen Festigkeit und der Resorption, der Fettgehalt des Ausgangsmaterials von wesentlichem Einfluss ist. Bevorzugt wird daher ein Unterhautgewebe mit einem Fettgehalt der trockenen Haut von nicht mehr als 2 % , besonders bevorzugt von 0,1 % bis 2 %, weiter bevorzugt von 0,5 % bis 1,6 % verwendet.

Außerdem erwies es sich als vorteilhaft, anstelle der üblichen Sterilisation des Endproduktes durch γ-Strahlen eine Begasung mit Ethylenoxid durchzuführen, wodurch offensichtlich der normale Vernetzungsgrad der Kollagenfibrillen gesteigert werden kann.

Für eine besonders gute Vernetzung und Stabilisierung des Kollagen-Materials wird bevorzugt eine Plasmasterilisation mit Wasserstoffperoxid-Begasung vorgenommen.

In vorteilhafter Ausgestaltung des Verfahrens werden der basischen und oxidativen Aufreinigung der Schweinehaut Salzbehandlungsstufen mit Kochsalz und Natriumhydrogencarbonat vorgeschaltet, um eine besonders schonende Quellung der Haut zu erreichen. Eine im Stand der Technik, beispielsweise gemäß dem Dokument DD 233 785 A1, durchgeführte pneumatische Auflockerung des Kollagens wird vorliegende aus Gründen einer besonders schonenden Behandlung nicht vorgenommen.

Trotz der verbesserten Nassfestigkeit zeigten die erfindungsgemäß hergestellten Kollagen-Materialien im Tierversuch nach Implantation unter die Rückenhaut von Ratten im Vergleich mit zwei handelsüblichen Produkten darüber hinaus überraschenderweise zudem eine wesentlich kürzere Resorptionszeit.

Überraschenderweise führen die genannten Veränderungen im Herstellungsverfahren des Kollagen-Materials auch zu einer vergleichsweise verbesserten hämostyptischen Wirkung.

Bei den erfindungsgemäß durchgeführten Veränderungen zum Herstellungsprozess und der daraus resultierenden systematischen Überprüfung verschiedener Versuchsherstellungen im Tierversuch, kam es zu einem völlig unvorhersehbaren Ergebnis. Es zeigte sich nämlich, dass der pH-Wert der Kollagensuspension von entscheidender Bedeutung für die biologische Verträglichkeit des Kollagen-Materials ist. So waren Kollagen-Materialien mit einem pH-Wert von 7 bis 8 deutlich besser verträglich als parallel geprüfte handelsübliche Verkaufsprodukte, die pH-Werte zwischen 4 und 5 aufwiesen.

Bei den weiteren Entwicklungsarbeiten in dieser Richtung zeigte sich, dass Kollagen-Materialien mit pH-Werten zwischen 6,5 und 8,5, vorzugsweise von 7,2 bis 7,8, die Verträglichkeit dergestalt verbesserten, dass im Wundbereich keine vermehrte Sekretion (Serombildung) stattfand, die Hautwunden p.p. verheilten, ein rascher Wundverschluss stattfand und bei der histologischen Auswertung von Gewebeproben, die dem Implantationsort entnommen worden waren, physiologische und dem artgerechten Heilungsverlauf entsprechende Verhältnisse anzutreffen waren.

Nachstehend werden Beispiele für die Herstellung von Kollagen-Materialien nach dem erfindungsgemäßen Verfahren angegeben, die dann als Basis für Vergleichsversuche mit handelsüblichen Produkten verwendet wurden.

### Beispiel 1:

Gespaltene Schweinehäute werden einer Salzbehandlung mit Kochsalz und Natriumhydrogencarbonat unterzogen und durch eine 24-stündige basische Behandlung mit 2%iger Natronlauge und eine 30 minütige oxidative Behandlung mit 20%iger Wasserstoffperoxidlösung aufgereinigt. Dabei findet bevorzugt zwischen den einzelnen Behandlungsstufen jeweils eine Spülung mit Wasser statt.

Anschließend werden die Hautstücke mit 0,2 m Phosphorsäure auf einen pH-Wert von 2,5 bis 3,5 eingestellt, gespült und zerkleinert (gewolft). Der erhaltene Kollagenbrei wird nach Zwischenfrostung bei -28 ± 10 °C gemahlen und mit Wasser vermischt, so dass eine homogene Suspension mit einem Kollagengehalt von etwa 1 % entsteht.

Zunächst wird eine mit einem Phosphatpuffer, insbesondere einem Gemisch einer Di- und Trinatriumhydrogenphosphat-Lösung, auf pH 6,5 bis 7,8 eingestellte Lösung von Gentamicin-Sulfat innerhalb von 10 Minuten zudosiert.

Anschließend wird die gesamte Suspension von homogenisiertem Kollagen und Gentamicin-Sulfat mit dem Phosphatpuffer auf einen neutralen bis leicht basischen pH-Wert eingestellt (pH 6,5 bis 8,5) und mit destilliertem Wasser auf einen Feststoffgehalt von 0,5 % bis 2,5 % eingestellt.

Daraufhin wird die Suspension bei maximal 25 °C langsam weitergerührt bis die Fibrillierung einsetzt.

Danach wird die Suspension in formgebende Gefäße abgefüllt und lyophilisiert (gefriergetrocknet).

### Beispiel 2:

Gespaltene Schweinehäute werden einer Salzbehandlung mit Kochsalz und Natriumhydrogencarbonat unterzogen und durch eine 24-stündige basische Behandlung mit 2%iger Natronlauge und eine 30 minütige oxidative Behandlung mit 20%iger Wasserstoffperoxidlösung aufgereinigt. Dabei findet bevorzugt zwischen den einzelnen Behandlungsstufen jeweils eine Spülung mit Wasser statt.

Anschließend werden die Hautstücke mit 0,2 m Phosphorsäure auf einen pH-Wert von 2,5 bis 3,5 eingestellt, gespült und zerkleinert (gewolft). Der erhaltene Kollagenbrei wird nach Zwischenfrostung bei -28 ± 10 °C gemahlen und mit Wasser vermischt, so dass eine homogene Suspension mit einem Kollagengehalt von etwa 1 % entsteht.

Zunächst wird der Suspension eine saure Lösung (pH 4,5 bis 5,5) von Gentamicin-Sulfat innerhalb von 10 Minuten zudosiert.

Anschließend wird die gesamte Suspension von homogenisiertem Kollagen und Gentamicin-Sulfat mit einem Phosphatpuffer, insbesondere einem Gemisch einer Di- und Trinatriumhydrogenphosphat-Lösung, auf einen neutralen bis leicht basischen pH-Wert eingestellt (pH 6,5 bis 8,5).

Daraufhin wird die Suspension mit destilliertem Wasser auf einen Feststoffgehalt bezüglich Kollagen von 0,5 % bis 2,5 % (üblicherweise 1 %) eingestellt.

Anschließend wird die Suspension bei maximal 25 °C langsam weitergerührt bis die Fibrillierung einsetzt.

Danach wird die Suspension in formgebende Gefäße abgefüllt.

Zur Verstärkung der Vernetzung wird der Brei mit einer geeigneten Vorrichtung in Lyophilisierschalen eingerüttelt und zur Vervollständigung der Fibrillierung mindestens 20 Stunden bei ca. 10 °C stehen gelassen.

Nach erfolgreicher Fibrillierung wird die Suspension lyophilisiert (gefriergetrocknet).

### Beispiel 3:

Gespaltene Schweinehäute werden einer Salzbehandlung mit Kochsalz und Natriumhydrogencarbonat unterzogen und durch eine 24-stündige basische Behandlung mit 2%iger Natronlauge und eine 30 minütige oxidative Behandlung mit 20%iger Wasserstoffperoxidlösung aufgereinigt. Dabei findet bevorzugt zwischen den einzelnen Behandlungsstufen jeweils eine Spülung mit Wasser statt.

Anschließend werden die Hautstücke mit 0,2 m Phosphorsäure auf einen pH-Wert von 2,5 bis 3,5 eingestellt, gespült und zerkleinert (gewolft). Der erhaltene Kollagenbrei wird nach Zwischenfrostung bei -28 ± 10 °C gemahlen und mit Wasser vermischt, so dass eine homogene Suspension mit einem Kollagengehalt von etwa 1 % entsteht.

Eine wäßrige 2%ige Gentamicin-Sulfatlösung wird über eine Kolonne mit phosphatbeladenem Anionenaustauscherharz geleitet bis im Eluat kein Sulfat mehr nachweisbar ist. Die so gewonnene Gentamicin-Phosphat-Lösung wird danach mit einem Phosphatpuffer, insbesondere einem Gemisch einer Di-und Trinatriumhydrogenphosphat-Lösung, auf einen pH-Wert von 6,5 bis 7,8 eingestellt and anschließend innerhalb von 20 bis 30 Minuten zur Kollagensuspension zudosiert.

Daraufhin wird die gesamte Suspension von homogenisiertem Kollagen und Gentamicin-Sulfat mit dem Phosphatpuffer auf einen neutralen bis leicht basischen pH-Wert eingestellt (pH 6,5 bis 8,5).

Anschließend wird die Suspension mit destilliertem Wasser auf einen Feststoffgehalt bezüglich Kollagen von 0,5 % bis 2,5 % (üblicherweise 1 %) eingestellt.

Danach wird die Suspension bei maximal 25 °C langsam weitergerührt bis die Fibrillierung einsetzt und in formgebende Gefäße abgefüllt.

Da in der Suspension keine störenden Sulfationen mehr vorhanden sind, ist eine Verstärkung der Vernetzung wie im Beispiel 2 beschrieben nicht mehr notwendig.

Nach erfolgreicher Fibrillierung wird die Suspension lyophilisiert (gefriergetrocknet).

Zur Beurteilung der Eigenschaften und Wirkungen wurde ein erfindungsgemäß hergestelltes Kollagen-Material mit zwei im Handel von unterschiedlichen Herstellern erhältlichen Produkten in Tierversuchen an Ratten verglichen, nämlich
1. erfindungsgemäß, insbesondere nach Beispiel 2, hergestelltes Kollagen-Material, insbesondere Kollagen-Vlies:
   Schweine-Kollagen,
   pH-Wert 7,6,
   Gentamicingehalt 1,3 mg Base pro cm²
2. Handelsprodukt A:
   Pferde-Kollagen,
   pH-Wert 4 bis 5,
   Gentamicingehalt 1,3 mg Base pro cm²
3. Handelsprodukt B:
   Pferde-Kollagen,
   pH-Wert 4 bis 5,
   Gentamicingehalt 1,3 mg Base pro cm²

Bei jeweils fünf Ratten pro Versuchszeitraum (5, 15, 35 Tage) wurden pro Tier zwei Kollagen-Material-Proben von jeweils 1 cm² Größe rechts und links paravertebral unter die Rückenhaut implantiert. Der Heilungsverlauf wurde täglich durch Adspektion überprüft. Nach Ablauf der Versuchszeiten wurden die Tiere getötet, vom Implantationsort die entsprechende Stelle samt Haut, Unterhaut, Kollagen-Material und ggf. Hautmuskulatur entnommen und diese histologisch (lichtmikroskopisch) untersucht.

### Handhabung

Das erfindungsgemäß hergestellte Kollagen-Material entsprach den Handhabungsvorgaben der Chirurgen, d.h. keine Haftung an Instrumenten, Handschuhen etc., bei fester Haftung auf der Wundoberfläche und sicherer sowie einfacher Applikation bei guter Nassfestigkeit. Bei den Vergleichsprodukten A und B wurden die bekannten Handhabungsnachteile (Haftung an Instrumenten und Handschuhen, deutlich geringere Nassfestigkeit) beobachtet.

### Blutstillung

Die bei dem erfindungsgemäß hergestellten Kollagen-Material beobachtete sehr gute Blutstillung ist auf die starke Fibrillierung während der Herstellung zurückzuführen. Im Vergleich zu einem speziell für die Blutstillung zugelassenen Handelsprodukt wurde die Gerinnungszeit auf ein Drittel bis ein Fünftel reduziert.

### Histologie

Das erfindungsgemäß hergestellte Kollagen-Material zeigt im Vergleich zu den Handelsprodukten A und B eine deutlich bessere Verträglichkeit. Zellen waren bereits nach 5 Tagen bis ins Zentrum der Implantate eingewandert, während bei den Vergleichsprodukten die Implantate höchstens marginal besiedelt waren.

Es wurden keine Zelltrümmer und keine nekrotischen Zellen (wie bei A und B) im Implantat selbst beobachtet.

Nach 15 Tagen war das erfindungsgemäß hergestellte Kollagen-Material teilweise resorbiert in das umliegende Bindegewebe integriert. Die Produkte A und B waren noch makroskopisch erkennbar und nicht nennenswert resorbiert.

Nach 35 Tagen waren bei dem erfindungsgemäß hergestellten Implantat nur noch marginale Reste histologisch erkennbar. Bei den Implantaten A und B waren die Kollagen-Materialien noch größtenteils erhalten.

### Heilungsverlauf

Auch im unmittelbar postoperativen Heilungsverlauf (Verträglichkeit) wurden deutliche Unterschiede gesehen. So kam es bei den Produkten A und B zu Schwellungen, die bei dem erfindungsgemäß hergestellten Kollagen-Material nicht beobachtet wurden. Weiterhin waren bei diesem Kollagen-Material nach 5 Tagen die Wunden p.p. fest verheilt, während bei den Produkte A und B die Wundränder nur verklebt waren und sich bei leichtem Druck aufspreizten. Darunter lagen auch die intakten Implantate, während im Gegensatz hierzu bei dem erfindungsgemäß hergestellten Kollagen-Material die Implantate makroskopisch nicht mehr erkennbar waren.

Die beschriebenen Versuchsergebnisse zeigen eindeutig, dass sowohl die Resorptionszeit als auch insbesondere die histologische Verträglichkeit der verschiedenen Kollagen-Material-Implantate nicht oder nur unwesentlich vom Antibiotikumgehalt beeinflusst werden, sondern dass möglicherweise die Art des Kollagens, insbesondere aber der wesentliche Unterschied im pH-Wert von ausschlaggebender Bedeutung für die deutlich bessere Verträglichkeit, die gute hämostyptische Wirkung und die stark verkürzte Resorptionszeit des erfindungsgemäß hergestellten Kollagen-Materials ist.

Die im Tierversuch beobachtete Förderung der Wundheilung durch das erfindungsgemäß hergestellte physiologische Kollagen-Material beruht auf einer Stimulation der Zellaktivität, insbesondere der Fibroblastenaktivität.

Bei ersten klinischen Anwendungen zeigte das erfindungsgemäß hergestellte Kollagen-Material im Vergleich zu bekannten Handelsprodukten erwartungsgemäß einen sehr viel besseren und rascheren Heilungsverlauf ohne Wundheilungsstörungen oder Serombildung.

## Patentansprüche

1. Verfahren zur Herstellung eines Kollagen-Materials, insbesondere eines Implantats zur Wundabdeckung, umfassend die Schritte:
- Herstellen einer Suspension, die ein telopeptid-haltiges Kollagen umfasst, wobei zur Herstellung der Suspension ein natives Kollagen verwendet wird,
- Zugabe eine Phosphatpuffers,
- Ruhenlassen der Suspension bis eine Verfestigung stattgefunden hat,
- Herstellung eines Flächengebildes durch Trockung des entstandenen Kollagen-haltigen Gemisches.

2. Verfahren zur Herstellung eines Kollagen-Materials nach dem vorstehenden Anspruch, **dadurch gekennzeichnet, dass** die Suspension unter Zugabe einer Säure zu einem Kollagen-haltigen Ausgangsmaterial verarbeitet wird.

3. Verfahren zur Herstellung eines Kollagen-Materials nach dem vorstehenden Anspruch, **dadurch gekennzeichnet, dass** Phosphorsäure verwendet wird.

4. Verfahren zur Herstellung eines Kollagen-Materials nach einem der vorstehenden Ansprüche, **dadurch gekennzeichnet, dass** als Kollagenmaterial Häute von Säugetieren, insbesondere von Schweinen verwendet werden.

5. Verfahren zur Herstellung eines Kollagen-Materials nach einem der vorstehenden Ansprüche, **dadurch gekennzeichnet, dass** das Kollagen-haltige Gemisch, insbesondere die Suspension durch Zugabe des Phosphatpuffers auf einen pH-Wert zwischen 5,5 und 8,5, bevorzugt zwischen 5,8 und 7,8, besonders bevorzugt zwischen 6,0 und 7,0 eingestellt wird.

6. Verfahren zur Herstellung eines Kollagen-Materials nach einem der vorstehenden Ansprüche, **dadurch gekennzeichnet, dass** die Suspension mit einem Kollagengehalt von 0,3 bis 7, vorzugsweise von 0,5 bis 3 und besonders bevorzugt 0,6 bis 1,5 Gewichts-% hergestellt wird.

7. Verfahren zur Herstellung eines Kollagen-Materials nach einem der vorstehenden Ansprüche, **dadurch gekennzeichnet, dass** das Gemisch vor der Trockung auf einen Feststoffgehalt von 0,2 bis 10 %, bevorzugt 0,3 bis 8 % und besonders bevorzugt 0,5 bis 2,5 % eingestellt wird.

8. Verfahren zur Herstellung eines Kollagen-Materials nach einem der vorstehenden Ansprüche, **dadurch gekennzeichnet, dass** ein Flächengebilde mit einem Phosphat-, insbesondere Dinatriumhydrogenphosphatanteil von 80 bis 500, vorzugsweise von 100 bis 400, besonders bevorzugt von 220 bis 370 mg/g hergestellt wird.

9. Verfahren zur Herstellung eines Kollagen-Materials nach einem der vorstehenden Ansprüche, **dadurch gekennzeichnet, dass** die Suspension mechanisch denaturiert wird, insbesondere mit einer Kolloidmühle.

10. Verfahren zur Herstellung eines Kollagen-Materials nach einem der vorstehenden Ansprüche, **dadurch gekennzeichnet, dass** ein Antibiotikum zugesetzt wird.

11. Kollagen-haltiges Flächengebilde, insbesondere hergestellt mit einem Verfahren nach einem der vorstehenden Ansprüche, umfassend ein natives, telopeptid-haltiges Kollagen, wobei der pH-Wert des Flächengebildes mehr als 5,8, vorzugsweise mehr als 6,5 und besonders bevorzugt mehr als 7,2 beträgt und wobei das Flächengebilde einen Phosphatanteil zwischen 80 und 500 mg/g aufweist.

12. Kollagen-haltiges Flächengebilde nach einem der vorstehenden Ansprüche, **dadurch gekennzeichnet, dass** das Flächengebilde nassstabil und trocken biegefest ist.

13. Kollagen-haltiges Flächengebilde nach einem der vorstehenden Ansprüche, **dadurch gekennzeichnet, dass** der Anteil an telopeptid-haltigem Kollagen am gesamten Kollagen mehr als 40 %, vorzugsweise mehr als 60 % und besonders bevorzugt mehr als 80 % beträgt.

## Claims

1. Process for producing a collagen material, in particular an implant for covering a wound, comprising the steps of:
- producing a suspension which comprises a telopeptide-containing collagen, wherein a native collagen is used for producing the suspension
- adding a phosphate buffer,
- allowing the suspension to rest until solidification has taken place,
- producing a planar formation by drying the resultant collagen-containing mixture.

2. Method for producing a collagen material as claimed in the preceding claim, **characterised in that** the suspension is processed into a collagen-containing starting material through the addition of an acid.

3. Method for producing a collagen material as claimed in the preceding claim, **characterised in that** phosphoric acid is used.

4. Method for producing a collagen material as claimed in any one of the preceding claims, **characterised in that** skins from mammals, in particular from pigs, are used as the collagen material.

5. Method for producing a collagen material as claimed in any one of the preceding claims, **characterised in that** the collagen-containing mixture, in particular the suspension is adjusted by addition of the phosphate buffer to a pH value between 5.5 and 8.5, preferably between 5.8 and 7.8, particularly preferably between 6.0 and 7.0.

6. Method for producing a collagen material as claimed in any one of the preceding claims, **characterised in that** the suspension is produced with a collagen content of 0.3 to 7, preferably 0.5 to 3 and particularly preferably 0.6 to 1.5 wt.%.

7. Method for producing a collagen material as claimed in any one of the preceding claims, **characterised in that**, prior to drying, the mixture is adjusted to a solids content of 0.2 to 10%, preferably 0.3 to 8% and particularly preferably 0.5 to 2.5%.

8. Method for producing a collagen material as claimed in any one of the preceding claims, **characterised in that** a planar formation is produced having a phosphate proportion, in particular a disodium hydrogen phosphate proportion of 80 to 500, preferably 100 to 400, particularly preferably 220 to 370 mg/g.

9. Method for forming a collagen material as claimed in any one of the preceding claims, **characterised in that** the suspension is mechanically denatured, in particular with a colloid mill.

10. Method for producing a collagen material as claimed in any one of the preceding claims, **characterised in that** an antibiotic is added.

11. Collagen-containing planar formation, in particular produced by a method as claimed in any one of the preceding claims, comprising a native, telopeptide-containing collagen, wherein the pH value of the planar formation is more than 5.8, preferably more than 6.5 and particularly preferably more than 7.2 and wherein the planar formation comprises a phosphate proportion between 80 and 500 mg/g.

12. Collagen-containing planar formation as claimed in any one of the preceding claims, **characterised in that** the planar formation is stable when wet and resistant to bending when dry.

13. Collagen-containing planar formation as claimed in any one of the preceding claims, **characterised in that** the proportion of telopeptide-containing collagen in the entire collagen is more than 40%, preferably more than 60% and particularly preferably more than 80%.

## Revendications

1. Procédé de production d'un matériau de collagène, en particulier d'un implant pour le pansement d'une plaie, comprenant les étapes consistant à :
- préparer une suspension qui comprend un collagène contenant un télopeptide, un collagène natif étant utilisé pour la préparation de la suspension,
- ajouter un tampon phosphate,
- laisser reposer la suspension jusqu'à ce qu'une solidification se produise,
- préparer une structure textile plane par séchage du mélange obtenu contenant du collagène.

2. Procédé de production d'un matériau de collagène selon la revendication précédente, **caractérisé en ce que** la suspension est transformée en une matière première contenant du collagène par l'addition d'un acide.

3. Procédé de production d'un matériau de collagène selon la revendication précédente, **caractérisé en ce que** de l'acide phosphorique est utilisé.

4. Procédé de production d'un matériau de collagène selon l'une des revendications précédentes, **caractérisé en ce que**, comme matériau de collagène, de la peau de mammifères, en particulier de porcs, est utilisée.

5. Procédé de production d'un matériau de collagène selon l'une des revendications précédentes, **caractérisé en ce que** le mélange contenant du collagène, en particulier la suspension, est ajusté à un pH situé entre 5,5 et 8,5, de préférence entre 5,8 et 7,8, en particulier préférentiellement entre 6,0 et 7,0 par l'addition du tampon de phosphate.

6. Procédé de production d'un matériau de collagène selon l'une des revendications précédentes, **caractérisé en ce que** la suspension est produite avec une teneur en collagène de 0,3 à 7, de préférence 0,5 à 3 et en particulier préférentiellement de 0,6 à 1,5 % en poids.

7. Procédé de production d'un matériau de collagène selon l'une des revendications précédentes, **caractérisé en ce que** le mélange est ajusté avant séchage à une teneur en matières solides de 0,2 à 10 %, de préférence 0,3 à 8 % et en particulier préférentiellement de 0,5 à 2,5 %.

8. Procédé de production d'un matériau de collagène selon l'une des revendications précédentes, **caractérisé en ce qu'**une structure textile plane ayant une proportion en phosphate, en particulier en dihydrogénophosphate de sodium, de 80 à 500, de préférence de 100 à 400, en particulier préférentiellement de 220 à 370 mg/g, est produite.

9. Procédé de production d'un matériau de collagène selon l'une des revendications précédentes, **caractérisé en ce que** la suspension est dénaturée mécaniquement, en particulier par un broyeur colloïdal.

10. Procédé de production d'un matériau de collagène selon l'une des revendications précédentes, **caractérisé en ce qu'**un antibiotique est ajouté.

11. Structure textile plane contenant du collagène, produite en particulier par un procédé selon l'une des revendications précédentes, comprenant un collagène natif, contenant un télopeptide, le pH de la structure textile plane atteignant plus de 5,8, de préférence plus de 6,5 et en particulier préférentiellement plus de 7,2, et la structure textile plane présentant une proportion de phosphate située entre 80 et 500 mg/g.

12. Structure textile plane contenant du collagène selon l'une des revendications précédentes, la structure textile plane étant **caractérisée en ce qu'**elle est stable au mouillage et résistante à la flexion à sec.

13. Structure textile plane contenant du collagène selon l'une des revendications précédentes, **caractérisée en ce que** la proportion de collagène contenant un télopeptide est de plus de 40 %, de préférence de plus de 60 % et en particulier préférentiellement de plus de 80 % par rapport au total du collagène.
